# EUROPEAN PATENT SPECIFICATION

(11) **EP 0 773 025 B1**
(45) Date of publication and mention of the grant of the patent: **07.06.2000**
(21) Application number: 96901349.9
(22) Date of filing: 26.01.1996
(51) Int. Cl.: A61K 31/44, A61K 9/50

(54) **NEW STABLE GALENIC FORMULATIONS CONTAINING AN ACID-LABILE BENZIMIDAZOL COMPOUND, AND PRODUCTION PROCESS**
NEUE STABILE GALENISCHE FORMULIERUNGEN, DIE EINE SÄURELABILE BENZIMIDAZOLVERBINDUNG ENTHALTEN, UND HERSTELLUNGSVERFAHREN
NOUVELLES FORMULATIONS GALENIQUES STABLES COMPRENANT UN COMPOSE DE BENZIMIDAZOLE ACIDE-LABILE, ET PROCEDE DE PRODUCTION

(30) Priority: 01.02.1995 ES 9500181
(43) Date of publication of application: 14.05.1997
(60) Divisional application: 99116334.6
(73) Proprietor: LABORATORIOS DEL DR. ESTEVE, S.A., 08041 Barcelona (ES)
(72) Inventor: BALLESTER RODES, Montserrat, E-08023 Barcelona (ES); VAN BOVEN, Marinus, E-08391 Tiana (ES)
(74) Representative: SUGRANES - VERDONCES - FERREGÜELA
(86) International application number: PCT/ES96/00013
(87) International publication number: WO 96/23500

(56) References cited:
- EP-A- 0 519 144
- EP-A- 0 519 365
- US-A- 5 045 321
- Handbook of Chemistry and Physics, 52nd Ed., 1971-1972, pp. B-106, B-107, B-150

## Description

### Field of the invention

The present invention is related to a new stable pharmaceutical preparation for oral administration containing a 2 [(2-pyridyl)methylsulphinyl]-benzimidazole derivative (hereinafter referred to as "benzimidazole compound") of formula I: 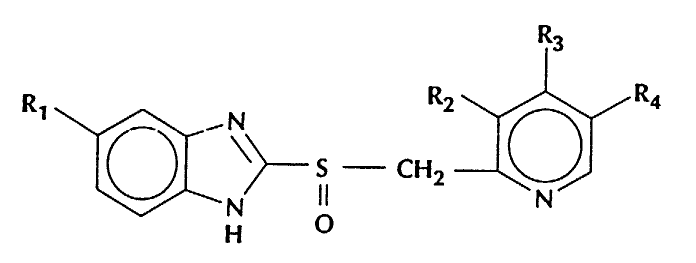 wherein R₁ is methoxy, R₂ is methyl, R₃ is methoxy, R₄ is methyl.

The invention also relates to a method for the manufacture of such preparation and to a method for the treatment of gastrointestinal diseases.

### Background of the invention

The above benzimidazole compound is a very effective drug for the treatment of gastric and duodenal ulcers, gastroesophageal reflux disease, severe erosive esophagitis, Zollinger-Ellison syndrome and H-pylori eradication. However, it is well known that this compound has poor stability. In the solid state it is susceptible to heat, moisture and light, and in aqueous solution or suspension its stability decreases with decreasing pH. The degradation of this compound is catalyzed by acidic reacting compounds.

Pharmaceutical preparations containing acid-labile compounds have to be subcoated in order to avoid a reaction between the active ingredient and the outer acidic enteric coating which reaction -if occurring- would result in degradation, destabilisation and consequently discolouration of the active ingredient.

The use of a barrier layer to protect the pharmaceutical from degradation caused by an enteric coating is well known from the prior art. Nevertheless, it is not possible to use conventional enteric coatings in a conventional way for acid labile benzimidazole compounds since decomposition takes place and the preparations become discoloured and lose in the active ingredient content with time. Prior art partially avoids the above mentioned stability problem by including an alkaline salt form of the benzimidazole compound or incorporating an alkaline reacting compound into an enteric coated preparation (magnesium oxide, hydroxide or carbonate, aluminium hydroxide, aluminium, calcium, sodium or potassium carbonate, phosphate or citrate, composite aluminium/magnesium compounds, sodium lauryl sulfate, aminoacids, N-methyl-D-glucamine, etc.) as described in US-A-4,786,505, US-A-5,232,706, EP-A-237200, EP-A-124495, US-A-5,385,739, EP-A-519144, the alkaline reacting compound being present within or on the surface of the nucleus together with the benzimidazole compound. Some authors use the alkaline reacting compound also in the composition of a second isolation layer to ensure stability of these forms. It is important to note that patent US-A-4,786,505, in its Example 1, Table 1 No.1, illustrates a formulation which is free of such alkaline compound and it is shown in Table 3 (No. 1-II) that this formulation has a rather poor stability. Thus, the association of an alkaline substance to the neutral form of the benzimidazole compound is tought in order to improve the stability of the active compound, especially for solid dosage forms, and enteric coating is recommended. That is, according to the state of the art, the addition of an alkaline substance to the pharmaceutical preparation is required to ensure the stability of the drug for long term storage.

### Outline of the invention

According to the present invention a high stability solid preparation containing the benzimidazole compound of formula I is obtained. The new galenic formulation do not contain alkaline reacting compounds; thus, an alkaline reacting compound is not present in the enteric coated preparation of the invention. Surprisingly, the obtained new preparation has a significantly enhanced stability for long-term storage, much higher than the known preparations, avoiding discolouration and loss of purity, thus being more suitable for pharmaceutical use.

The new preparation is characterized in that to an inert sugar/starch spherical core, a first layer is applied containing a mixture of the benzimidazole compound of formula I as active ingredient, hydroxypropylmethylcellulose as a water soluble inert polymer and talc as pharmaceutical acceptable excipient, followed by a second isolation layer formed by hydroxypropylmethylcellulose as water soluble polymer and titanium dioxide and talc as compatible excipients. Finally a third layer consisting of an enteric coating is applied. The core, the process conditions and the excipients have been selected in order to obtain the required coating efficiency for each layer.

The resulting new preparation is resistant to dissolution in acid media being stable for passage through the gastric juice, and dissolves rapidly in a neutral to alkaline media, the conditions in the proximal part of the small intestine. In fact, the acid resistance, tested as per US Pharmacopoeia, demonstrated that after 2 hours the total amount of the benzimidazole remained intact and that upon changing the pH to 6,8, after 30 minutes all the benzimidazole was dissolved (tested as per US Pharmacopoeia).

### Detailed description of the invention

In a fluidized bed apparatus, uniform spherical inert cores (composition as per US Pharmacopoeia) are coated with a first layer consisting of the acid labile benzimidazole compound, hydroxy-propylmethylcellulose and talc. The second layer consists of hydroxypropylmethylcellulose, talc and titanium dioxide as a pigment. The third and enteric coating layer consists of an enteric coating polymer formed by co-polymerized methacrylic acid / methacrylic acid methyl esters, a plasticizer such as triethylcitrate or similar plasticizers, and talc.

The layers are applied by conventional fluidized bed coating techniques using aqueous solutions or dispersions.

The active ingredient can be administered in the same dosages and according to the same protocol as the corresponding already marketed commercial dosage forms.

For oral administration, the final dosage may take the form of capsules containing the pellets, or pellets compressed into a tablet.

The dose as the benzimidazole compound lies within the range of about 1 mg to 100 mg/kg/day, adjusted to individual patients needs and for as long as clinically indicated.

The invention is described in detail in the following example:

### Example

In 3440 g of deionized water 436 g of omeprazole (I; R₁=-OCH₃, R₂=CH₃, R₃=-OCH₃, R₄=CH₃), 444 g of hydroxypropylmethylcellulose and 118 g of talc are dispersed.

3010 g of inert uniform sugar/starch spheres (composition according to US Pharmacopoeia) are introduced into a fluidized bed apparatus and the previous obtained dispersion is sprayed on the spheres. After spraying, the spheres are dried before applying the second layer.

In 2365 g of deionized water, 355 g of hydroxy-propylmethylcellulose, 43 g of talc and 43 g of titanium dioxide are dispersed and the resulting aqueous dispersion is sprayed on the spheres obtained in the previous step. After spraying, the spheres are dried before applying the third enteric coating layer.

In 1890 g of deionized water, 1950 g of methacrylic acid copolymer (US Pharmacopoeia, type C aqueous dispersion), 98 g of triethylcitrate and 98 g of talc are dispersed, and the resulting aqueous dispersion is sprayed on the spheres obtained in the previous step. After applying this final enteric coating layer the spheres (pellets) are dried.

The pellets thus obtained were stored in closed polyethylene bags within a closed cardboard fibre container and also in closed glass containers and submitted to so called accelerated conditions, that is 40°C and 75% relative humidity. At the same time pellets obtained from Prilosec® capsules (Merck/Astra trademark) were stored in identical containers and submitted to the same conditions. The results of the test under accelerated conditions are summarized in tables 1, 2 and 3. They demonstrate a superior stability over the already authorized product on the market.

**TABLE 1**

| **COLOR OF THE PELLETS** | | | |
|---|---|---|---|
| | AT THE START | 1 MONTH | 3 MONTHS |
| Pellets (I) - Fiber container | A | A | D |
| Pellets (I) - Glass container | A | A | B |
| | | | |
| Prilosec - Fiber container | A | C | F |
| Prilosec - Glass container | A | A | E |
| | | | |
| A : White | C : faint brown | | E : brown |
| B : Pinkish white | D : light brown | | F : Deep brown |

**TABLE 2**

| **OMEPRAZOLE PURITY *** | | | |
|---|---|---|---|
| | AT THE START | 1 MONTH | 3 MONTHS |
| Pellets (I) - Fiber container | 99,5% | 98,8% | 52% |
| Pellets (I) - Glass container | 99,5% | 98,7% | 97,9% |
| | | | |
| Prilosec - Fiber container | 96,1% | 85,2% | 1% |
| Prilosec - Glass container | 96,1% | 96,2% | 1% |

| | | | |
|---|---|---|---|
| * Analyzed as per HPLC, described in Pharmaeuropa, Vol. 4, n° 2, June 1992 and expressed as direct area percentage. | | | |

**TABLE 3**

| **OMEPRAZOLE RECOVERY AFTER US DISSOLUTION TEST** | | |
|---|---|---|
| | 1 MONTH | 3 MONTHS |
| Pellets (I) - Fiber container | 96,8% | 9,2% |
| Pellets (I) - Glass container | 99,9% | 73,8% |
| | | |
| Prilosec - Fiber container | 21,3% | << 1% |
| Prilosec - Glass container | 84,5% | << 1% |

### Biopharmaceutical studies

The purpose of the study was to investigate the bioavailability and pharmacokinetic profile of the newly developed formulation of omeprazole in comparison with the standard capsule formulation (Prilosec®; 20 mg).

Hard gelatin capsules were filled with the new galenic form of omeprazole, prepared according to example 1, in an amount corresponding to 20 mg of omeprazole.

The experimental design was a single center, open-label, randomized, 2-way cross-over study in 24 healthy male and female subjects.

Subjects reported to the clinical unit at about 8 p.m. in the evening prior to the day of treatment, and they remained hospitalized until 12 hours after drug intake. Subjects received a standard meal the evening before dosing.

The drug was given in the clinical unit with 200 ml of tap water after subjects had been fasting for at least 10 hours.

The concentration of omeprazole in blood plasma was assayed by a validated high pressure liquid chromatography method with UV detection (Internal Report No. CPR 95-742). The mean plasma concentrations are given in Table 4.

**TABLE 4**

| The mean plasma concentrations (ng/ml) after 20 mg oral doses of omeprazole new formulation given as capsules *vs.* Prilosec® capsules. | | |
|---|---|---|
| Time (h.) | New formulation | Prilosec® |
| Baseline | 0,0 | 0,0 |
| 0,5 | 16,4 | 6,3 |
| 1,0 | 103,7 | 105,4 |
| 1,5 | 161,8 | 191,9 |
| 2,0 | 192,0 | 210,1 |
| 2,5 | 165,4 | 168,4 |
| 3,0 | 132,7 | 119,8 |
| 3,5 | 103,8 | 87,6 |
| 4,0 | 81,4 | 63,5 |
| 5,0 | 39,7 | 47,2 |
| 6,0 | 14,9 | 22,0 |
| 7,0 | 8,1 | 9,5 |
| 8,0 | 5,4 | 5,7 |
| 12,0 | 0,0 | 2,3 |

The pharmacokinetic results for omeprazole were comparable with those reported in the literature (Wilde MI, McTavish D. Omeprazole. An update of its pharmacology and therapeutic use in acid related disorders. Drugs 1994, 48: 91-132). The arithmetic mean (SD) half-lives of elimination of omeprazole were 0,9 (0,4) and 1,1 (0,7) h after oral administration of the new and Prilosec® formulation, respectively. The arithmetic mean (SD) Tₘₐₓ values of omeprazole were 2,3 (1,0) and 2,0 (1,1) h after administration of the new and Prilosec® formulation, respectively. The corresponding values for the geometric mean of the maximum plasma concentration Cₘₐₓ were 249 (197) and 241 (174) ng/ml, and those of AUC_{0-∞} were 434 (440) and 486 (436) ng h/ml, respectively.

The ratios of geometric means (new/Prilosec®) of Cₘₐₓ and AUC were 1,03 in both cases, and the 2-side 90% confidence intervals (CI) for these ratios were entirely within the interval 0,80 - 1,25. According to the CPMP guidance for bioequivalence studies, bioequivalence of the formulations (new formulation and Prilosec® formulation) can be accepted (References: CPMP Working Party on the Efficacy of Medicinal Products 1991. Note for guidance: Investigation of bioavailability and bioequivalence; Schulz HU, Steinijans VW. Striving for standards in bioequivalence assessment: A review; Int. J. Clin. Pharmacol. Ther. Toxicol. 1992, 30 (suppl.1): S1-S6).

Thus, by preparing omeprazole capsules according to the present invention, it is possible to obtain a preparation with the same bioavailability as the Prilosec® capsules containing the same amount of micronized active compound.

## Claims

1. A stable oral pharmaceutical preparation containing an acid labile benzimidazole compound of formula I: 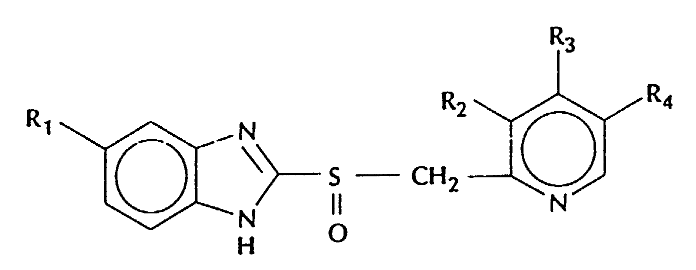 wherein R₁ is methoxy, R₂ is methyl, R₃ is methoxy, R₄ is methyl, which comprises:
(a) a nucleus formed by coating a spherical inert core with the acid labile benzimidazole, hydroxy-propylmethylcellulose and talc;
(b) an inert coating disposed on said nucleus, formed by hydroxypropylmethylcellulose, titanium dioxide and talc;
(c) an outer layer disposed on the previous coating comprising an enteric coating containing co-polymerized methacrylic acid/methacrylic acid methyl ester, triethylcitrate and talc.

2. A process for the preparation of a stable oral pharmaceutical preparation containing an acid labile benzimidazole compound of formula I as defined in claim 1 as active ingredient, which comprises: preparing a nucleus formed by spraying a layer that contains the acid labile benzimidazole, hydroxypropylmethylcellulose and talc on an inert core in a fluidized bed apparatus, drying, coating said nucleus by spraying an inert layer formed by hydroxypropylmethylcellulose, titanium dioxide and talc, drying, and finally coating by spraying an enteric coating containing co-polymerized methacrylic acid methyl ester, triethyl citrate and talc, and drying.

3. A galenic preparation in the form of capsules or tablets containing the stable oral pharmaceutical preparation according to claim 1.

## Patentansprüche

1. Stabile orale pharmazeutische Zubereitung, enthaltend eine säurelabile Benzimidazolverbindung der Formel I 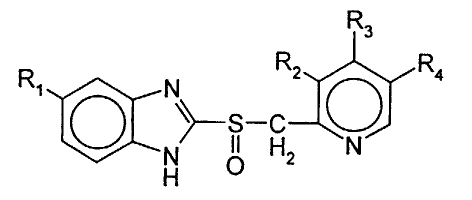 in der
R₁ eine Methoxygruppe ist,
R₂ eine Methylgruppe ist,
R₃ eine Methoxygruppe ist,
R₄ eine Methylgruppe ist,
umfassend:
a) einen Kern erzeugt durch Überziehen eines inerten sphärischen Kens mit dem säurelabilen Benzimidazol, Hydroxypropylmethylcellulose und Talk;
b) einen inerten Überzug, auf diesem Kern, erzeugt durch Kern erzeugt durch Hydroxypropylmethylcellulose, Titandioxid und Talk;
c) eine äussere Schicht auf dem vorherigen Überzug umfassend einen magensaftresistenten Überzug, der co-polymerisierte Methacrylsäure / Methacrylsäuremethylester, Triethylcitrat und Talk enthält.

2. Verfahren zur Herstellung einer stabilen oralen pharmazeutischen Zubereitung enthaltend die säurelabile Benzimidazolverbindung der Formel I gemäss Anspruch 1 als Wirkstoff, umfassend:
Herstellen eines Kerns durch Aufsprühen einer Schicht, die diese säurelabile Benzimidazolverbindung, Hydroxypropylmethylcellulose, und Talk enthält, auf einen inerten Kern in einer Wirbelschichtapparatur, Trocknen, Überziehen dieses Kerns durch Aufsprühen einer inerten Schicht, erzeugt aus Hydroxypropylmethylcellulose, Titandioxid und Talk, Trocknen und schliesslich Überziehen durch Aufsprühen eines magensaftresistenten Überzugs, enthaltend co-polymerisierten Methacrylsäuremethylester, Triethylcitrat un Talk, und Trocknen.

3. Galenische Zubereitung in Form von Kapseln oder Tabletten, enthaltend die stabile orale pharmazeutische Zubereitung nach Anspruch 1.

## Revendications

1. Composition pharmaceutique stable orale contenant un composé de benzimidazole labile sous l'action d'un acide, ledit composé répondant à la formule I: 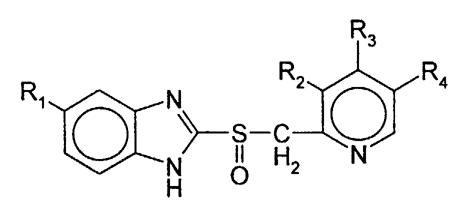 où R₁ est méthoxy, R₂ est méthyle, R₃ est méthoxy et R₄ est méthyle, ladite composition comprenant :
(a) un noyau formé par revêtement d'une âme sphérique inerte avec du benzimidazole labile sous l'action d'un acide, de l'hydroxypropylméthylcellulose et du talc ;
(b) un revêtement inerte disposé autour dudit noyau et formé par de l'hydroxypropylméthylcellulose, du dioxyde de titane et du talc ;
(c) une couche externe disposée autour du revêtement précédent et comprenant un revêtement entérique contenant un matériau co-polymérisé d'acide méthacrylique/méthacrylate de méthyle, du citrate de triéthyle et du talc.

2. Procédé pour la préparation d'une composition stable orale contenant un composé de benzimidazole de formule I labile sous l'action d'un acide tel que défini dans la revendication 1 en tant qu'ingrédient actif, ledit procédé comprenant : la préparation d'un noyau formé par projection d'une couche qui contient ledit composé benzimidazole labile sous l'action d'un acide, l'hydroxypropylméthylcellulose et du talc sur une âme inerte dans un appareil à lit fluidisé, le séchage, le revêtement dudit noyau par projection d'une couche inerte formée par de l'hydroxypropylméthylcellulose, du dioxyde de titane et du talc, séchage puis finalement revêtement par projection d'un revêtement entérique contenant un matériau co-polymérisé d'acide méthacrylique/méthacrylate de méthyle, du citrate de triéthyle et du talc, et séchage.

3. Préparation galénique sous la forme de gélules ou de comprimés contenant la composition stable orale suivant la revendication 1.
